# EUROPEAN PATENT APPLICATION

(11) **EP 4 579 234 A1**
(43) Date of publication of application: **02.07.2025**
(21) Application number: 23848979.3
(22) Date of filing: 12.05.2023
(51) Int. Cl.: G01N 30/02, G01N 30/34, G01N 30/32, G01N 30/54, G01N 30/74

(54) **METHOD FOR ANALYZING RELATED SUBSTANCES IN BIOTINOYL TRIPEPTIDE-1 PREPARATION**

(30) Priority: 02.08.2022 CN 202210921653
(71) Applicant: Zhaoke (Guangzhou) Ophthalmology Pharmaceutical Limited, Guangzhou, Guangdong 511462 (CN)
(72) Inventor: XIE, Zhijun, Guangzhou, Guangdong 511462 (CN); TAN, Ping, Guangzhou, Guangdong 511462 (CN)
(74) Representative: Bayramoglu et al.
(86) International application number: PCT/CN2023/093879
(87) International publication number: WO 2024/027268

(57) **Abstract**

A method for analyzing related substances in a fequesetide preparation. In the method, high-performance liquid chromatography is used for determination, where the chromatographic column used therein is a YMC-Triart C18 chromatographic column, 4.6 × 150 mm, 3 µm; the column temperature is 35°C; the sample size is 100 mL; the detection wavelength is 210 nm; the mobile phase A is a 0.1% aqueous trifluoroacetic acid; the mobile phase B is 0.1% trifluoroacetic acid-acetonitrile; and gradient elution is used, and the elution program is as follows: at 0 min: the volume ratio of the mobile phase A to the mobile phase B is 100:0; at 8-13 min: the volume ratio of the mobile phase A to the mobile phase B is 85:15; and from 13 min to 20 min: the volume ratio of the mobile phase A to the mobile phase B is 100:0. In the present analysis method, the degree of separation between a main peak and impurity peaks is good, the peak pattern, tailing and number of theoretical plates are good, and the detection time is short; therefore, an effective method is provided for formulating quality standards for products of this type.

## Description

### TECHNICAL FIELD

The present disclosure relates to the field of analytical chemistry, and more specifically to a method for analyzing related substances in a fequesetide preparation.

### BACKGROUND ART

Fequesetide corresponds to the actin-binding core functional domain of thymosin beta-4 (Tβ4), discovered by scientists at the National Institutes of Health (NIH), comprising seven amino acids with the sequence: Leucine-Lysine-Lysine-Threonine-Glutamic acid-Threonine-Glutamine (L-K-K-T-E-T-Q). Research findings indicate that fequesetide exhibits multiple biological functions, including but not limited to, binding to actin, promoting hair follicle growth, stimulating vascular regeneration (angiogenesis), accelerating wound healing, and enhancing cellular migration.

Results from pharmacodynamic studies and safety assessment trials demonstrate that the fequesetide is safe and non-toxic, exhibits efficacy in promoting corneal epithelial repair in rats, and mitigates inflammatory responses. The fequesetide ophthalmic solutions are indicated for the treatment of: corneal epithelial punctate defects associated with dry eye syndrome; and corneal epithelial lamellar defects caused by various etiological factors (including first-degree and second-degree burns, mechanical injuries, and iatrogenic causes).

During the synthesis of the fequesetide, epimers, deletion peptides, insertion peptides, and other impurities may be generated. Specifically, a total of 7 single-site epimers are obtained (where the diastereoisomer of Thr is the D-allo structure), along with 6 easily generated deletion peptide impurities, 13 insertion peptide impurities, 7 misjoined peptide impurities, and 10 cleaved peptide impurities. Additionally, based on the sequence structure and synthesis process, the impurity IM020/Glu7, which is prone to formation through degradation, is synthesized. In total, 44 impurities are studied.

At present, the Chinese Pharmacopoeia does not include a detection method for active pharmaceutical ingredients, and the detection method provided by the fequesetide manufacturer, Zhejiang Peptites Biotech Co., Ltd, is as follows:
Chromatographic column: YMC-PackODS-AQ 4.6×250 mm, 3 µm (C18 high-performance chromatographic column manufactured by YMC Co., Ltd., Japan)
Column temperature: 45 °C
Mobile phase A: 50 mM phosphate buffer (pH6.0); Mobile phase B: 50% Methanol
Flow rate: 0.8 mL/min
Injection volume: 20.0 µL (2 mg/mL)
Detection wavelength: UV 210 nm
Elution gradient:

| **Time (min)** | **Mobile phase A (%)** | **Mobile phase B (%)** |
|---|---|---|
| 0 | 98 | 2 |
| 30 | 92 | 8 |
| 40 | 40 | 60 |
| 50 | 40 | 60 |
| 51 | 98 | 2 |
| 60 | 98 | 2 |

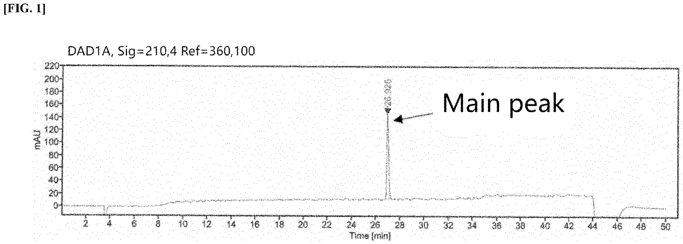

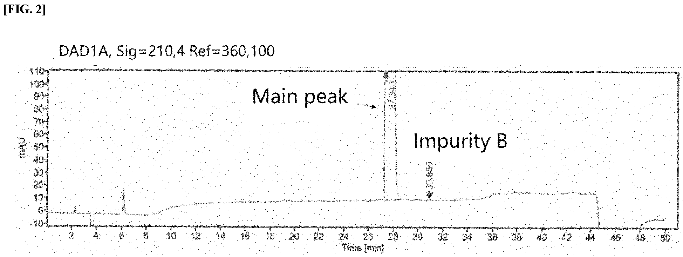

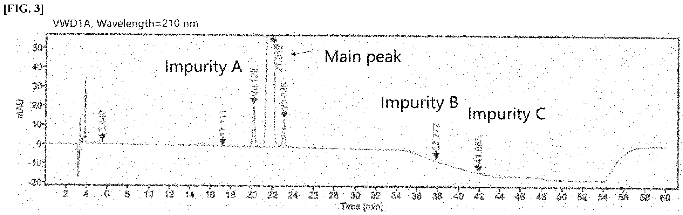

As shown in FIGS. 1-3, FIG. 1 shows a chromatogram of fequesetide detection in the prior art; FIG. 2 shows a chromatogram of the active pharmaceutical ingredient (API) and impurity A (Glu⁷-fequesetide) in the fequesetide according to the prior art; and FIG. 3 illustrates a chromatogram of the API with impurity B (D-Leu¹-fequesetide) and impurity C (Di-Glu⁵-fequesetide) in the prior art. The problems with the above detection method are as follows: the detection time is 50 min or longer, which affects the detection efficiency. Additionally, the specificity for detecting multiple impurities is not satisfactory, and the resolution between the impurity peak and the main peak is inadequate. After detecting the mixed standard, the resolution of the first two impurities is very poor (far less than 1.5), and the impurities eluting after the main peak fail to achieve baseline separation. As a result, it is not possible to simultaneously detect multiple known impurities. Therefore, a new purity detection method is needed to detect and separate the multiple known impurities described above and other unknown impurities.

Hence, it is necessary to develop an analytical detection method capable of effectively separating related substances in the fequesetide while eliminating interference from excipients.

### CONTENT OF THE INVENTION

To address the impurities in the fequesetide, the inventors of the present disclosure have developed multiple liquid chromatography conditions for separation. Simultaneously, through the study of the process, a process capable of fully controlling these impurities has been ultimately determined. The present disclosure has optimized the detection methods for several common impurities.

The present disclosure provides a method capable of efficiently analyzing related substances in the fequesetide, with a significantly reduced analysis time. The analytical method comprises a sample preparation step and a detection process performed by high-performance liquid chromatography (HPLC) method; and the HPLC method employs a chromatographic column packed with octadecylsilyl-bonded silica gel as a stationary phase.

Further, the analytical method is capable of effectively separating the fequesetide from related impurities. Preferably, the related substances refer to Glu⁷-fequesetide (impurity A), D-Leu¹-fequesetide (impurity B), and Di-Glu⁵-fequesetide (impurity C).

Further, the mobile phase for the HPLC method comprises mobile phase A and mobile phase B, where the mobile phase A is an aqueous solution of trifluoroacetic acid, with a volume ratio of trifluoroacetic acid to water ranging from 0.05:100 to 0.15:100, for example, the volume ratio may also be 0.06:100, 0.07:100, 0.08:100, 0.09:100, 0.10:100, 0.11:100, 0.12:100, 0.13:100, or 0.14:100. Preferably, the volume ratio of trifluoroacetic acid to water in the mobile phase A is 0.1:100.

Further, the mobile phase B is trifluoroacetic acid-acetonitrile, where a volume ratio of trifluoroacetic acid to acetonitrile ranges from 0.05:100 to 0.15:100, for example, the volume ratio may also be 0.06:100, 0.07:100, 0.08:100, 0.09:100, 0.10:100, 0.11:100, 0.12:100, 0.13:100, or 0.14:100. Preferably, the volume ratio of trifluoroacetic acid to acetonitrile in the mobile phase B is 0.1:100.

Further, a detection wavelength for the HPLC method ranges from 200 nm to 220 nm, for example, the detection wavelength may also be 201 nm, 202 nm, 203 nm, 204 nm, 205 nm, 206 nm, 207 nm, 208 nm, 209 nm, 210 nm, 211 nm, 212 nm, 213 nm, 214 nm, 215 nm, 216 nm, 217 nm, 218 nm, or 219 nm. Preferably, the detection wavelength is 210 nm.

Further, a column temperature of the chromatographic column is maintained in the range of 30-40°C, for example, the column temperature of the chromatographic column may also be 31°C, 32°C, 33°C, 34°C, 35°C, 36°C, 37°C, 38°C, or 39°C. Preferably, the temperature of the chromatographic column is 35°C.

Further, an injection volume for the HPLC method ranges from 90 µL to 110 µL, for example, the injection volume may also be 91 µL, 92 µL, 93 µL, 94 µL, 95 µL, 96 µL, 97 µL, 98 µL, 99 µL, 100 µL, 101µL, 102 µL, 103 µL, 104 µL, 105 µL, 106 µL, 107 µL, 108 µL, or 109 µL. Preferably, the injection volume is 100 µL.

Further, a flow rate for the HPLC method ranges from 0.8 mL/min to 1.5 mL/min, for example, the flow rate may be 0.8 mL/min, 0.9 mL/min, 1.0 mL/min, 1.1 mL/min, 1.2 mL/min, 1.3 mL/min, 1.4 mL/min, or 1.5 mL/min. Preferably, the flow rate is 0.8 mL/min.

Preferably, a specification of the chromatographic column is 4.6 × 150 mm, 3 µm.

Further, the chromatographic column is one selected from a Waters column, an Agilent column, a Thermo column, a Phenomenex column, a Shimadzu column, and a YMC column. In a specific embodiment, the chromatographic column is a YMC-Triart C18 column.

Further, the HPLC method employs gradient elution, where the gradient elution program is as follows:
at 0 min: the volume ratio of mobile phase A to mobile phase B is 100:0;
at 8 min: the volume ratio of mobile phase A to mobile phase B ranges from 83:17 to 87:13;
at 8 min-13 min: the volume ratio of mobile phase A to mobile phase B is maintained in a range of 83:17 to 87:13; and
from 13 min to 20 min: the volume ratio of mobile phase A to mobile phase B is 100:0.

Preferably, the gradient elution program is as follows:
at 0 min: the volume ratio of mobile phase A to mobile phase B is 100:0;
at 8 min: the volume ratio of mobile phase A to mobile phase B is maintained at 85:15;
from 8 min to 13 min: the volume ratio of mobile phase A to mobile phase B is maintained at 85:15; and
from 13 min to 20 min: the volume ratio of mobile phase A to mobile phase B is 100:0.

In a specific embodiment, the elution program is as follows:

| Time (min) | Mobile phase A (%) | Mobile phase B(%) |
|---|---|---|
| 0 | 100 | 0 |
| 8 | 85 | 15 |
| 13 | 85 | 15 |
| 13.1 | 100 | 0 |
| 20 | 100 | 0 |

Further, in certain embodiments requiring quantification, the method further comprises calculating the peak area by integration.

A second aspect of the present disclosure provides a use of the analytical method as described above in the quality control of the fequesetide preparation.

In some specific embodiments, the use includes the detection of the purity of the fequesetide, the analysis of the types of impurities and/or the purity of the impurities in the fequesetide.

In a specific embodiment, the fequesetide preparation is a fequesetide ophthalmic solution.

The method described in the present disclosure employs a chromatographic column packed with amino acid/polypeptide-modified octadecylsilyl-bonded silica gel (4.6 × 150 mm, 3 µm) as a stationary phase, and the mobile phases are: A: ultrapure water (0.1% trifluoroacetic acid); and B: acetonitrile (0.1% trifluoroacetic acid); detection wavelength: 210 nm, column temperature: 35°C, flow rate: 0.8-1.5 mL/min. The method is capable of effectively detecting and separating three known impurities of the fequesetide (Glu7-fequesetide, D-Leu¹-fequesetide, and Di-Glu⁵-fequesetide) to avoid interference, furthermore, the detection time is reduced to approximately 20 min, thereby enabling the establishment of a quality standard for the fequesetide.

### DESCRIPTION OF THE DRAWINGS

FIG. 1 shows a chromatogram of fequesetide detection according to the prior art;
FIG. 2 shows a chromatogram of the active pharmaceutical ingredient (API) and related substances in fequesetide, according to the prior art;
FIG. 3 shows a chromatogram of the API and other related substances in fequesetide according to the prior art;
FIG. 4 shows a chromatogram under optimized conditions of Example 1;
FIG. 5 shows a chromatogram of isocratic elution in Example 2;
FIG. 6 shows a chromatogram after reducing injection volume in Example 3; and
FIG. 7 shows a chromatogram after increasing the gradient elution in Example 4.

### DETAILED DESCRIPTION

The present disclosure discloses a method for analyzing related substances in a fequesetide preparation, and those skilled in the art may refer to the content of the present disclosure and make appropriate modifications to the process parameters to achieve the desired results. It should be particularly pointed out that such similar substitutions and modifications would be readily apparent to those skilled in the art, and are all considered to be included in the present disclosure. The method of the present disclosure has been described by preferred examples, and it would be readily apparent to those skilled in the art that the methods and uses described herein may be modified, appropriately changed, or combined without departing from the scope, spirit, and content of the present disclosure to implement and apply the technology of the present disclosure.

Unless otherwise defined herein, all scientific and technical terms used in the present disclosure shall be construed to have meanings that are commonly understood and conventionally used by persons of ordinary skill in the art to which the present disclosure pertains.

In the present disclosure, the term "mobile phase" refers to a substance in the chromatographic process that carries analyte components and propels the sample forward while maintaining equilibrium with the stationary phase.

To enable those skilled in the art to better understand the technical solution of the present disclosure, the present disclosure is further described in detail below in conjunction with specific embodiments.

In the following examples, the fequesetide was obtained from Zhejiang Peptites Biotech Co., Ltd, with the batch number of PT2021122101, the water used was ultrapure water, and all other reagents were of analytical grade.

### Example 1:

Afequesetide ophthalmic solution is prepared according to the following method:
1) approximately 70% of the prescribed amount of water for injection was added into a preparation tank of appropriate capacity;
2) a prescribed amount of inorganic salts was added into a stainless steel barrel, followed by the addition of approximately 15% of the prescribed amount of water for injection, and the mixture was stirred until dissolved; subsequently, a prescribed amount of the fequesetide was added, and the mixture was stirred to obtain a clear solution;
3) the mixed solution was transferred into a 25 L preparation tank, followed by rinsing the container containing the mixed solution three times with approximately 10% of the prescribed amount of water for injection, with the rinse solutions being collected and combined into the preparation tank, the combined solution was stirred for 5 min to achieve uniform mixing;
4) an appropriate amount of hydrochloric acid was slowly added to cooled water for injection to prepare a 1 mol/L hydrochloric acid solution for subsequent use;
5) with stirring initiated, the prepared hydrochloric acid solution was slowly added while stirring continuously until the pH was adjusted to 6.5-7.5, and the stirring was continued for 5 min; and
6) the water for injection was supplemented to reach the full prescribed amount, and stirring was initiated for 10 min to achieve uniform mixing, followed by sampling for testing.

Chromatographic column was packed with octadecylsilane-bonded silica gel as the stationary phase.

Chromatographic conditions were as follows:
[Instrument] Agilent Infinity II
[Detector] Agilent DAD
Mobile phase A: 0.1% aqueous trifluoroacetic acid
Mobile phase B: 0.1% trifluoroacetic acid in acetonitrile
Chromatographic column: YMC-Triart C18, 4.6 × 150 mm, 3 µm
Column temperature: 35 °C, Injection volume: 100 µL, Detection wavelength: 210 nm, and Flow rate: 0.8 mL/min
Gradient elution is performed according to Table 1.

**Table 1 Elution program**

| Time (min) | Mobile phase A (%) | Mobile phase B (%) |
|---|---|---|
| 0 | 100 | 0 |
| 8 | 85 | 15 |
| 13 | 85 | 15 |
| 13.1 | 100 | 0 |
| 20 | 100 | 0 |

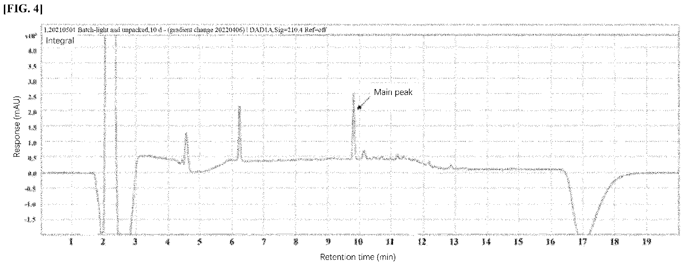

As shown in FIG. 4, the resolution between the main peak and the impurity peak in the fequesetide according to the method is excellent (2.5), and the peak shape, tailing factor (1.1), and theoretical plate number (13000) are synergistically optimized through the process, moreover, the injection time is significantly reduced to 20 minutes, and the main peak has a well-defined peak shape, minimal tailing factor, and excellent resolution from impurity peaks.

### Example 2: Changing the Chromatographic Column and Adjusting the Elution Gradient to Isocratic Elution Based on the Original Chromatographic Conditions

Chromatographic conditions were as follows:
Mobile phase A: 0.1% aqueous trifluoroacetic acid
Mobile phase B: 0.1% trifluoroacetic acid in acetonitrile
Chromatographic column: Agilent Zorbax SB-C18 chromatographic column, 4.6 × 150 mm, 5 µm
Column temperature: 35°C, Injection volume: 100 µL, Detection wavelength: 210 nm, and Flow rate: 0.8 mL/min

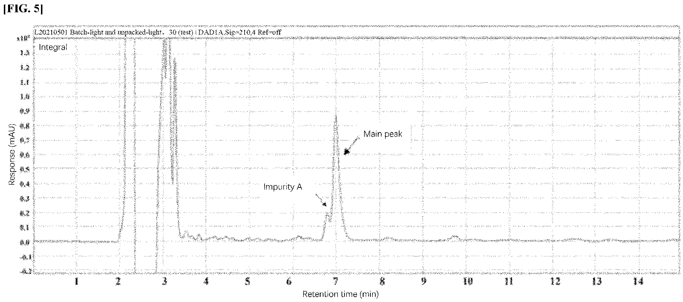

The elution gradient was adjusted to isocratic elution (mobile phase A:B = 90:10), and the elution was maintained for 30 min The elution profile of the main peak was monitored under isocratic elution conditions, where the organic phase concentration was maintained at 10%. As shown in FIG. 5, under the three gradient conditions, impurity peaks are observed at the elution positions of the main peak of the fequesetide, with relatively low resolution. It is experimentally demonstrated that under isocratic elution conditions, the resolution between the main peak and impurity peaks is poor, and under conditions of low organic phase ratio, the main peak exhibits severe tailing.

### Example 3: Reducing the Injection Volume to 50 µL and Further Evaluating the Elution Gradient

Chromatographic conditions were as follows:
Mobile phase A: 0.1% aqueous trifluoroacetic acid
Mobile phase B: 0.1% trifluoroacetic acid in acetonitrile
Chromatographic column: YMC-Triart C18, 4.6 × 150 mm, 3 µm
Column temperature: 35°C, Injection volume: 50 µL, Detection wavelength: 210 nm; Flow rate: 0.8 mL/min

During the initial 3 min of the elution, polar excipient peaks were eluted under a high aqueous phase ratio, between 3 and 15 min, the main peak was eluted with a 7% organic phase, the peak shape of the main peak and the separation of the main peak from impurity peaks were monitored to ensure that the elution of the main peak was not affected by gradient variations. Gradient elution was performed according to Table 2.

**Table 2 Elution program**

| Time (min) | Mobile phase A (%) | Mobile phase B (%) |
|---|---|---|
| 0 | 100 | 0 |
| 3 | 98 | 2 |
| 3.1 | 93 | 7 |
| 15 | 93 | 7 |
| 15.1 | 85 | 15 |
| 20 | 85 | 15 |
| 20.1 | 100 | 0 |
| 30 | 100 | 0 |

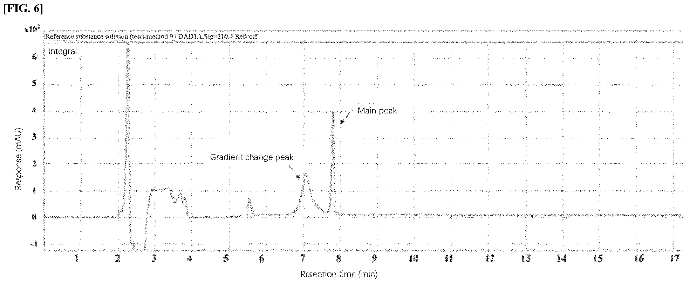

As shown in FIG. 6, the main peak is significantly interfered by the gradient change peak.

### Example 4: Increasing Gradient Ramp Rate and Monitoring Separation of Main Peak from Impurity Peaks

Chromatographic conditions were as follows:
Mobile phase A: 0.1% aqueous trifluoroacetic acid
Mobile phase B: 0.1% trifluoroacetic acid in acetonitrile
Chromatographic column: YMC-Triart C18, 4.6 × 150 mm, 3 µm
Column temperature: 35 °C, Injection volume: 100 µL, Detection wavelength: 210 nm, and Flow rate: 0.8 mL/min

The gradient elution was carried out according to Table 3.

**Table 3 Elution program**

| Time (min) | Mobile phase A (%) | Mobile phase B (%) |
|---|---|---|
| 0 | 98 | 0 |
| 8 | 85 | 15 |
| 13 | 85 | 15 |
| 13.1 | 100 | 0 |
| 20 | 100 | 0 |

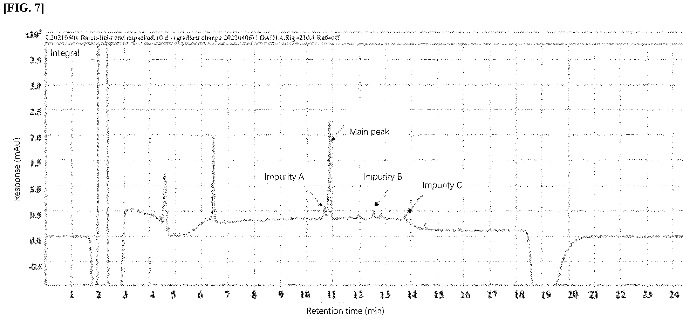

As shown in FIG. 7, the resolution (1.1) between the main peak and impurity peaks of the fequesetide in the test sample solution is unsatisfactory.

The examples described above are merely preferred examples of the present disclosure and are not intended to limit the present disclosure. For those skilled in the art, the present disclosure may be subjected to various modifications and variations. Any modifications, equivalent substitutions, improvements, and equivalents made within the spirit and principles of the present disclosure shall be included within the scope of protection of the present disclosure.

## Claims

1. A method for analyzing related substances in a fequesetide preparation, comprising the following steps:
(1) preparing a sample; and
(2) detecting with a high-performance liquid chromatography (HPLC) method; wherein the HPLC method employs a chromatographic column packed with octadecylsilyl-bonded silica gel as a stationary phase; a mobile phase for the HPLC method comprises mobile phase A and mobile phase B, wherein the mobile phase A is an aqueous solution of trifluoroacetic acid, with a volume ratio of trifluoroacetic acid to water ranging from 0.05:100 to 0.15:100, and the mobile phase B is trifluoroacetic acid-acetonitrile.

2. The method for analyzing related substances in the fequesetide preparation according to claim 1, wherein in the mobile phase B, a volume ratio of trifluoroacetic acid to acetonitrile ranges from 0.05:100 to 0.15:100.

3. The method for analyzing related substances in the fequesetide preparation according to claim 1, wherein a column temperature of the chromatographic column ranges from 30°C to 40°C.

4. The method for analyzing related substances in the fequesetide preparation according to claim 1, wherein an injection volume for the HPLC method ranges from 90 µL to 110 µL.

5. The method for analyzing related substances in the fequesetide preparation according to claim 1, wherein a detection wavelength for the HPLC method is 210 nm.

6. The method for analyzing related substances in the fequesetide preparation according to claim 1, wherein the chromatographic column is one selected from a Waters column, an Agilent column, a Thermo column, a Phenomenex column, a Shimadzu column, and a YMC column.

7. The method for analyzing related substances in the fequesetide preparation according to claim 1, wherein a specification of the chromatographic column is 4.6 × 150 mm, 3 µm.

8. The method for analyzing related substances in the fequesetide preparation according to claim 1, wherein the HPLC method employs gradient elution, with the gradient elution program as follows:
at 0 min: the volume ratio of mobile phase A to mobile phase B is 100:0;
at 8 min: the volume ratio of mobile phase A to mobile phase B ranges from 83:17 to 87:13;
from 8 min to 13 min: the volume ratio of mobile phase A to mobile phase B ranges from 83:17 to 87:13; and
from 13 min to 20 min: the volume ratio of mobile phase A to mobile phase B is 100:0.

9. The method for analyzing related substances in the fequesetide preparation according to claim 1, wherein the HPLC method employs gradient elution, with the gradient elution program as follows:
at 0 min: the volume ratio of mobile phase A to mobile phase B is 100:0;
at 8 min: the volume ratio of mobile phase A to mobile phase B is 85:15;
from 8 min to 13 min: the volume ratio of mobile phase A to mobile phase B is 85:15; and
from 13 min to 20 min: the volume ratio of mobile phase A to mobile phase B is 100:0.

10. A use of the method for analyzing related substances in the fequesetide preparation according to any one of claims of 1-9 in quality control of the fequesetide preparation.
